# FASCICULE DE BREVET EUROPEEN

(11) **EP 1 901 688 B1**
(45) Date de publication et mention de la délivrance du brevet: **09.09.2009**
(21) Numéro de dépôt: 06764844.4
(22) Date de dépôt: 06.06.2006
(51) Int. Cl.: A61F 9/06

(54) **LUNETTES DE PROTECTION**
SCHUTZBRILLE
PROTECTIVE GLASSES

(30) Priorité: 07.06.2005 FR 0551522
(43) Date de publication de la demande: 26.03.2008
(73) Titulaire: COMMISSARIAT A L'ENERGIE ATOMIQUE, 75015 Paris (FR)
(72) Inventeur: DEMONCHY, Jean-François, F-33470 Gujan Mestras (FR)
(74) Mandataire: Poulin, Gérard
(86) Numéro de dépôt international: PCT/FR2006/050522
(87) Numéro de publication internationale: WO 2006/131671

(56) Documents cités:
- EP-A- 1 148 718
- US-A- 5 760 852
- PATENT ABSTRACTS OF JAPAN vol. 2000, no. 26, 1 juillet 2002 (2002-07-01) & JP 2001 259883 A (MINOLTA CO LTD), 25 septembre 2001 (2001-09-25)

## Description

### DOMAINE TECHNIQUE

L'invention concerne des lunettes de protection des yeux d'un opérateur situé à proximité d'une ou plusieurs sources de rayonnement lumineux, visible ou non, par exemple de type laser.

### ÉTAT DE LA TECHNIQUE ANTÉRIEURE

Le document EP 1 148 718 A décrit des lunettes de protection des yeux d'un opérateur situé à proximité d'une ou plusieurs source de rayonnement lumineux comportant:
- des lunettes de vision numérique;
- une caméra vidéo de déplacement pour visualiser l'environnement lors du déplacement;
- une caméra vidéo de réglage pour visualiser la zone de travail;
- un dispositif de contrôle qui comprend une alimentation

Dans le domaine des outils de protection des yeux à l'encontre de rayonnements lumineux, les fournisseurs de lunettes proposent des solutions qui sont des moyens d'atténuation ou de réflexion passifs de ces rayonnements, sans aucune possibilité d'évolution de ces lunettes en ce qui concerne la fourniture de prestations annexes.

De plus de telles solutions ne répondent pas totalement aux besoins de l'invention :
- Les lunettes de protection de l'art connu ne sont fournies que pour des plages de longueurs d'onde fixes.
- Ces lunettes de protection ne permettent pas de voir des faisceaux lasers émettant dans le domaine non visible. Elles imposent donc l'utilisation d'un faisceau laser d'alignement dans une longueur d'onde autre que celles des faisceaux lasers de travail.
- Ces lunettes de protection sont calibrées pour certaines puissances. Si celles-ci sont fortes cela empêche une bonne visibilité de l'environnement, qui est de plus faible intensité.
- Ces lunettes de protection, en réalisant une protection par réflexion, engendrent un risque pour les autres opérateurs, voire pour le matériel environnant.
- Ces lunettes de protection, même correctement calibrées, peuvent être endommagées par les faisceaux reçus ou par des rayures mécaniques. Elles deviennent alors potentiellement dangereuses car un tel endommagement n'est pas toujours perceptible. Un contrôle régulier de ces lunettes est donc nécessaire.
- Pour certaines puissances il n'existe pas de lunettes de protection efficaces.

L'objet de l'invention est de proposer une interface entre les yeux d'un opérateur et une source de rayonnement extérieur permettant d'assurer une protection totale de ces yeux, quelle que soit la longueur d'onde ou la puissance de ce rayonnement. Il n'est, toutefois, pas question de protéger les yeux de cet opérateur contre les effets d'un laser de très forte puissance, par exemple un laser de découpe.

### EXPOSÉ DE L'INVENTION

L'invention concerne des lunettes de protection des yeux d'un opérateur situé à proximité d'une ou plusieurs sources de rayonnement lumineux, par exemple de type laser comportant :
- des lunettes de vision numérique,
- au moins une caméra vidéo,
- un dispositif de contrôle, qui comprend une alimentation,
caractérisées en ce qu'elles comportent deux caméras vidéo commutables :
- une première caméra vidéo de déplacement, qui sert à visualiser l'environnement lors du déplacement,
- une seconde caméra vidéo de réglage, qui sert à visualiser la zone de travail.

Les lunettes de vision numérique peuvent être des lunettes de vision virtuelle à double moniteur LCD. La caméra vidéo peut être une caméra de type « pin-hole » en CCD, ou en CMOS.

Les lunettes de protection de l'invention peuvent être recouvertes d'un revêtement absorbant.

Dans une première variante de réalisation le dispositif de contrôle comprend également une unité d'émission vidéo apte à émettre un signal aller vers une unité de traitement à distance.

Dans une seconde variante de réalisation le dispositif de contrôle comprend également une unité de réception vidéo apte à recevoir un signal retour provenant d'une unité de traitement à distance et à envoyer ce signal vidéo traité vers les lunettes de vision numérique.

La première caméra vidéo de déplacement peut être une caméra, par exemple CCD, avec un grand angle de vision, une grand profondeur de champ, et fonctionnant dans le domaine visible.

La seconde caméra vidéo de réglage peut être une caméra, par exemple CCD, avec un angle de vision plus étroit que la première caméra, une profondeur de champ plus faible, et fonctionnant dans le domaine visible ou non.

Les lunettes de protection de l'invention présentent de nombreux avantages par rapport aux lunettes de protection de l'art connu :
- Elles ne fonctionnent pas en atténuation de l'intensité lumineuse, et ne perturbent pas la perception de l'environnement lors de déplacements, de recherche de matériel, de prise de notes, de lecture de procédures ou de toutes autres actions menées par l'opérateur, par exemple dans une salle laser.
- Elles restent efficaces quelle que soit la longueur d'onde et la puissance de la source de rayonnement. II ne peut donc plus y avoir d'erreur sur le choix de la protection à utiliser dans telle ou telle installation.
- Elles permettent à un opérateur de travailler dans un laboratoire utilisant plusieurs lasers de longueurs d'onde différentes.
- Elles permettent de visualiser certains lasers pulsés, en jouant sur la durée d'acquisition du capteur utilisé dans la caméra vidéo.
- Dans les cas de hauts flux, ces lunettes peuvent être recouvertes d'un revêtement absorbant, qui supprime tous les problèmes liés aux réflexions.
- En choisissant bien la technologie de la caméra, on peut visualiser l'environnement d'une source intense de lumière, permettant ainsi la perception par l'utilisateur d'une tache très lumineuse sur l'écran, mais une vue parfaite du reste de l'environnement. Tout phénomène d'éblouissement de l'oeil est donc complètement annulé.
- De telles lunettes ont un faible coût de fabrication et un faible poids.
- Elles permettent de travailler directement sur des lasers émettant dans des longueurs d'ondes invisibles à l'oeil humain sans passer par des lasers annexes d'alignement. La caméra vidéo permet de capter ces longueurs d'ondes, qui peuvent être restituées sur les écrans de visualisation des lunettes dans une couleur visible sans pour autant perturber totalement le reste du spectre vu.
- L'adjonction d'un système de transmission hertzien permet de suivre à distance les faits et gestes d'un opérateur. Une telle fonction ouvre de nombreuses possibilités dans le domaine de la sécurité
ou de la conservation des connaissances, par exemple.

### BRÈVE DESCRIPTION DES DESSINS

La figure 1 illustre schématiquement un mode de réalisation des lunettes de protection de l'invention.

Les figures 2, 3 et 4 illustrent trois variantes de réalisation des lunettes de protection de l'invention.

### EXPOSÉ DÉTAILLÉ DE MODES DE RÉALISATION PARTICULIERS

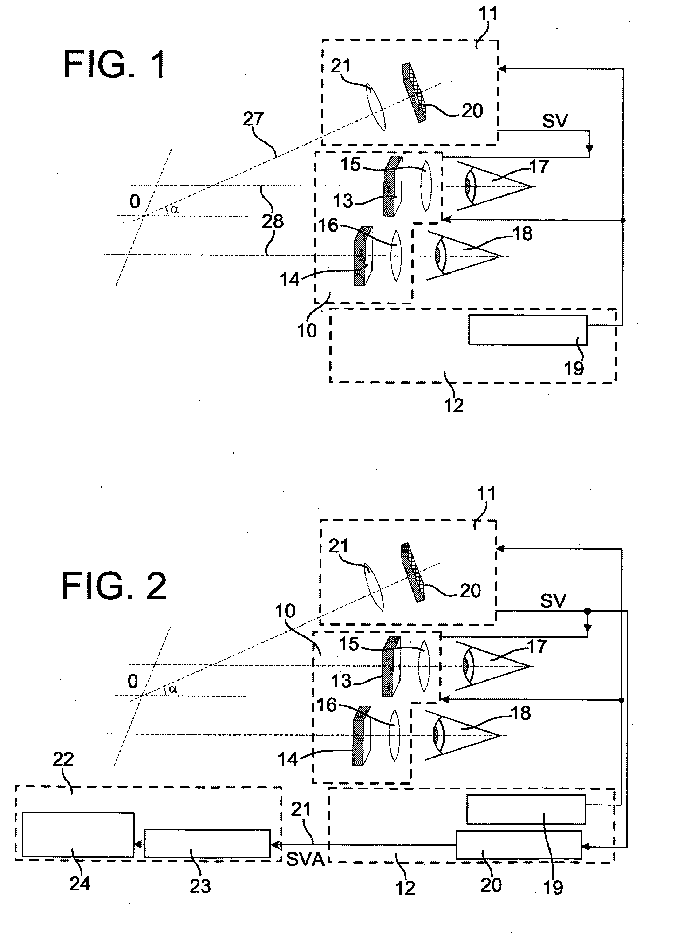

Comme illustré sur la figure 1, les lunettes de protection de l'invention comprennent :
- des lunettes de vision numérique 10 disposées devant les yeux 17 et 18 d'un opérateur,
- au moins une caméra vidéo 11 dirigée vers la scène à visualiser (point 0), et qui émet en sortie un signal vidéo SV vers ces lunettes 10,
- un dispositif de contrôle 12 comprenant l'alimentation 19 de ces lunettes 10 et de cette caméra 11, qui peut être disposé à la ceinture de l'opérateur.

Les lunettes 10 et la caméra vidéo sont, toutes deux, orientées vers un même point 0. L'angle α entre la direction perpendiculaire 27 à la cellule 20 de la caméra, et la direction perpendiculaire 28 aux écrans 13 et 14 des lunettes illustré sur la figure 1, est réglable en fonction de la distance de travail.

Dans ces lunettes 10, afin de simuler une vision en relief à la distance de travail, il faut que l'angle α soit non nul.

Les lunettes de vision numérique 10 sont des lunettes de vision virtuelle dédiées à l'origine à une utilisation ludique (console de jeux ou lecteur DVD portable).

Ces lunettes 10 comprennent donc un écran (13, 14) associé à une optique (15, 16) en face de chaque oeil (17, 18) de l'opérateur, chaque écran recevant le signal SV.

De telles lunettes 10 permettent une immersion dans une scène virtuelle.

La caméra vidéo 11 peut être une caméra CCD (« Charge Coupled Device ») comprenant un capteur CCD 20 qui se présente sous la forme d'une matrice de pixels, chacun de ces pixels produisant des électrons quand il reçoit des photons. Ici le capteur 20 est associé à une optique 21. La caméra vidéo 11 peut également être une caméra CMOS.

Dans une première variante de réalisation illustrée sur la figure 2, le dispositif de contrôle 12 comprend, outre l'alimentation 19, une unité d'émission vidéo 20 qui émet un signal vidéo aller SVA, par transmission hertzienne ou par câble (21), vers une unité de réception vidéo 23 et une unité de visualisation et d'enregistrement 24 situées dans une unité de traitement à distance 22.

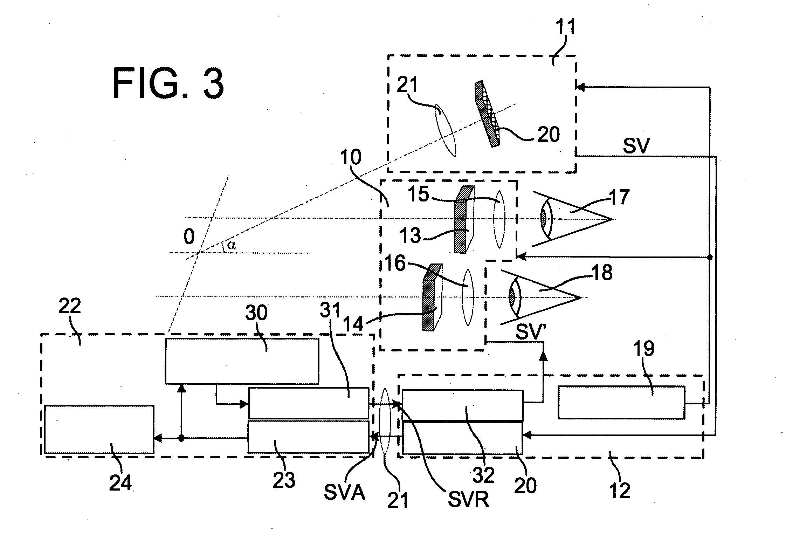

Dans une seconde variante de réalisation illustrée sur la figure 3, l'unité de traitement à distance 22 comprend, en outre des éléments illustrés sur la figure 2, une unité de traitement numérique de l'image en temps réel 30, une unité d'émission vidéo 31 qui émet un signal vidéo retour SVR. Le dispositif de contrôle 12 comprend une unité de réception vidéo 32 qui reçoit ce signal SVR et dont le signal de sortie SV' est envoyé vers les lunettes de vision numérique 10.

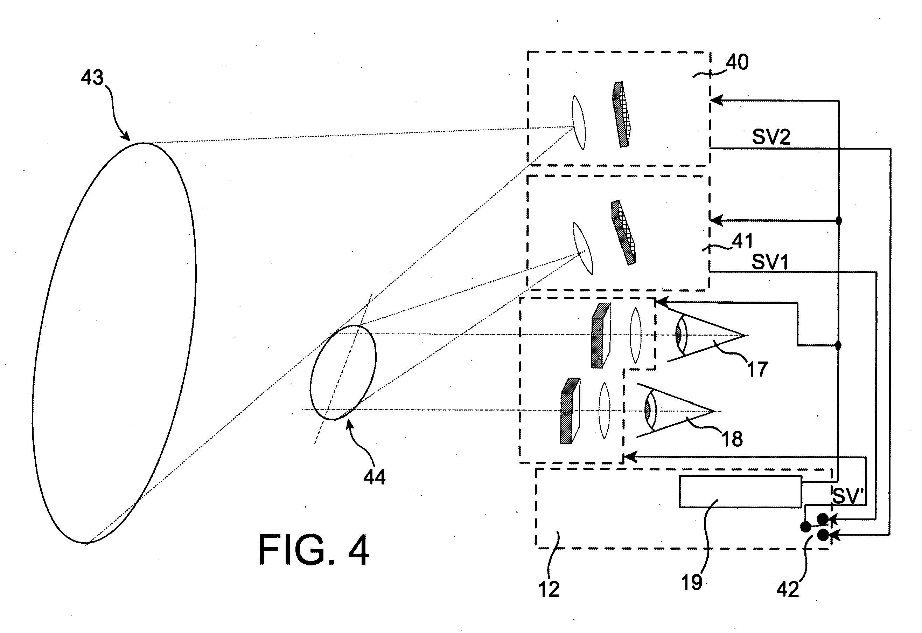

Comme illustré sur la figure 4, les lunettes de l'invention utilisent deux caméras vidéo commutables :
- une première caméra vidéo de déplacement 40 (grand angle de vision, grande profondeur de champ, capteur dans le domaine visible),
- une seconde caméra vidéo de réglage 41 (angle de vision plus étroit, profondeur de champ plus faible, capteur dans le domaine visible ou non).

La première caméra 40 sert à visualiser l'environnement lors du déplacement. La seconde caméra 41 sert à visualiser la zone de travail. Chaque caméra sort un signal vidéo (SV1, SV2). Sur la figure 4, l'ellipse 43 illustre le champ de visualisation des déplacements. L'ellipse 44 illustre le champ de visualisation de travail. Dans les lunettes, l'utilisateur peut passer, à l'aide d'un commutateur 42, d'une vue à l'autre.

La figure 4 n'illustre pas le fait que ce commutateur 42 permet d'alimenter la caméra utilisée et de couper l'autre caméra dans un souci de préserver la durée de vie de l'alimentation 19, par exemple constituée par des accumulateurs.

### Exemple de réalisation

Dans un exemple de réalisation les éléments constitutifs des lunettes de l'invention sont les éléments suivants.

Les lunettes de vision numérique 10 sont des lunettes numériques à deux écrans à cristaux liquides (LCD ou "liquid cristal display") d'une résolution de 180 000 pixels venant se placer devant chaque oeil de l'opérateur, et donc à double moniteur LCD, par exemple de type « RIMAX Virtuel Vision 2-0 ».

La caméra vidéo 11 est une caméra CCD à objectif « pin-hole », par exemple une caméra de type « Snake CCD Camera » de la société MISUMI (MS-C493A-3C-P01). Le positionnement de cette caméra est calculé pour avoir le maximum de précision à une distance correspondant à une distance de lecture (oeil/livre). Ce choix de la caractéristique « pin-hole » est fait pour obtenir une profondeur de champ maximale.

L'ensemble lunettes 10 + caméra 11 peut être recouvert d'un revêtement absorbant.

L'alimentation 19 de ces lunettes 10 et de cette caméra vidéo 11 est réalisée par des accumulateurs rechargeables qui peuvent être disposés à la ceinture de l'opérateur. Les lunettes de protection de l'invention sont donc totalement autonomes. Un tel ensemble de visualisation n'est ni plus encombrant, ni plus lourd que certaines lunettes de protection de l'art connu.

De telles lunettes de protection peuvent être utilisées notamment comme équipement de protection pour des personnels utilisant des lasers de classe supérieur à 2, des soudeurs à l'arc, des opérateurs travaillant aux alentours d'un four.

De manière générale, de telles lunettes de protection peuvent servir de protection à l'encontre de tout rayonnement lumineux pouvant porter préjudice à l'intégrité de l'oeil ou dans toutes zones où la possibilité d'éblouissement présente un risque.

De telles lunettes de protection permettent, de plus, la visualisation de longueurs d'ondes invisibles.

## Revendications

1. Lunettes de protection des yeux (17, 18) d'un opérateur situé à proximité d'une ou plusieurs sources de rayonnement lumineux comportant :
- des lunettes de vision numérique (10),
- au moins une caméra vidéo (11),
- un dispositif de contrôle (12), qui comprend une alimentation (19),
**caractérisées en ce qu'**elles comprennent deux caméras vidéos commutables :
- une première caméra vidéo de déplacement (40), qui sert à visualiser l'environnement lors du déplacement,
- une seconde caméra vidéo de réglage (41), qui sert à visualiser la zone de travail.

2. Lunettes selon la revendication 1, dans lesquelles les lunettes de vision numérique (10) sont des lunettes de vision virtuelle à double moniteur LCD.

3. Lunettes selon la revendication 1, dans lesquelles la caméra vidéo (11) est une caméra CCD.

4. Lunettes selon la revendication 1, dans lesquelles la caméra vidéo (11) est une caméra CMOS.

5. Lunettes selon la revendication 1, dans lesquelles la caméra vidéo (11) est une caméra « pin-hole ».

6. Lunettes selon la revendication 1, dans lesquelles la caméra vidéo (11) est une caméra équipé d'un objectif à optique standard.

7. Lunettes de protection selon la revendication 1, qui sont recouvertes d'un revêtement absorbant.

8. Lunettes de protection selon la revendication 1, dans lesquelles le dispositif de contrôle (12) comprend une unité d'émission vidéo (20) apte à émettre un signal vidéo aller (SVA) vers une unité de traitement à distance (22).

9. Lunettes de protection selon la revendication 8, dans lesquelles le dispositif de contrôle (12) comprend une unité de réception vidéo (32) apte à recevoir un signal vidéo retour (SVR) provenant de l'unité de traitement à distance (22) et à envoyer un signal vidéo (SV') vers les lunettes de vision numérique (10).

10. Lunettes de protection selon la revendication 1, dans lesquelles la première caméra vidéo de déplacement (40) est une caméra vidéo avec un grand angle de vision, une grand profondeur de champ, et fonctionnant dans le visible.

11. Lunettes de protection selon la revendication 1, dans lesquelles la seconde caméra vidéo de réglage (41) est une caméra vidéo avec un angle de vision plus étroit que la première caméra, une profondeur de champ plus faible, et fonctionnant dans une gamme de longueurs d'onde visible ou non.

12. Lunettes selon la revendication 1, dans laquelle la ou les sources de rayonnement sont une ou des sources de type laser.

## Claims

1. Eye protection glasses (17, 18) for an operator in the vicinity of one or more sources of light radiation, comprising:
- digital viewing glasses (10),
- at least one video camera (11),
- a command device (12), which comprises a power source (19),
**characterized in that** they comprise two switchable video cameras:
- a first video camera for movement (40) used to view surroundings when moving within the environment,
- a second video camera for adjustment (41), used to view the work area.

2. Glasses according to claim 1, in which the digital viewing glasses (10) are virtual viewing glasses with double LCD display.

3. Glasses according to claim 1, in which the video camera (11) is a CCD camera.

4. Glasses according to claim 1, in which the video camera (11) is a CMOS camera.

5. Glasses according to claim 1, in which the video camera (11) is a « pin-hole » camera.

6. Glasses according to claim 1, in which the video camera (11) is a camera equipped with a standard lens.

7. Glasses according to claim 1, which are coated with an absorbent coating.

8. Glasses according to claim 1, in which the command device (12) comprises a video transmission unit (20) able to transmit a go video signal (SVA) to a remote processing unit (22).

9. Glasses according to claim 8, in which the command device (12) comprises a video receiving unit (32) able to receive a return video signal (SVR) from the remote processing unit (22) and to send a video signal (SV') to the digital viewing glasses (10).

10. Glasses according to claim 1, in which the first video camera (40) for movement is a video camera with a wide view angle, large field depth and functioning in the visible range.

11. Glasses according to claim 1, in which the second video camera (41) for adjustment is a video camera with a view angle narrower than the first camera, a smaller field depth and functioning in the range of visible or invisible wavelengths.

12. Glasses according to claim 1, in which the source or sources of radiation are one or more sources of laser type.

## Patentansprüche

1. Schutzbrille für die Augen (17, 18) eines in der Nähe einer (oder mehrerer) Strahlungsquelle(n) befindlichen Operators, umfassend:
- digitale Sichtbrille bzw. -brillen (10),
- mindestens eine Videokamera (11),
- eine Kontrollvorrichtung (12), die eine Versorgung (19) umfasst,
**dadurch gekennzeichnet, dass** sie zwei kommutierbare Videokameras umfasst:
- eine Bewegungs- oder erste Kamera (40), die dazu dient, die Umgehung während der Bewegung zu visualisieren,
- eine Einstellungs- oder zweite Kamera (41), die dazu dient, die Arbeitszone zu visualisieren.

2. Brille nach Anspruch 1, bei der die digitale Sichtbrille bzw. -brillen (10) virtuelle Sichtbrillen mit LCD-Doppelmonitor sind.

3. Brille nach Anspruch 1, bei der die Videokamera (11) eine CCD-Kamera ist.

4. Brille nach Anspruch 1, bei der die Videokamera (11) eine CMOS-Kamera ist.

5. Brille nach Anspruch 1, bei der die Videokamera (11) eine Lochkamera ist.

6. Brille nach Anspruch 1, bei der die Videokamera (11) eine mit einem Standardoptik-Objektiv ausgerüstete Kamera ist.

7. Schutzbrille nach Anspruch 1, die mit einem absorbierenden Überzug beschichtet ist.

8. Schutzbrille nach Anspruch 1 bei der die Kontrollvorrichtung (12) eine Video-Sendeeinheit (20) umfasst, fähig ein Video-Hinsignal (SVA) in Richtung einer Distanzverarbeitungseinheit (22) zu senden.

9. Schutzbrille nach Anspruch 8, bei der die Kontrollvorrichtung (12) eine Video-Empfangseinheit (32) umfasst, fähig ein von der Distanzverarbeitungseinheit (22) kommendes Video-Rücksignal (SVR) zu empfangen und ein Videosignal (SV') zu der digitalen Sichtbrille (10) zu senden.

10. Schutzbrille nach Anspruch 1, bei der die Bewegungs- oder erste Kamera (40) eine Videokamera mit einem großen Sichtwinkel und einer großen Tiefenschärfe ist und im sichtbaren Bereich arbeitet.

11. Schutzbrille nach Anspruch 1, bei der die Einstellungs- oder zweite Kamera (41) eine Videokamera mit einem kleineren Sichtwinkel als die erste Kamera und einer kleineren Tiefenschärfe ist und in einem Bereich sichtbarer oder unsichtbarer Wellenlängen arbeitet.

12. Schutzbrille nach Anspruch 1 mit, als Strahlungsquelle(n), einer (oder mehreren) Laserquelle(n) .
